Europäisches Patentamt

European Patent Office

Office européen des brevets

(11) Numéro de publication: **0 431 989 A1**

# (12) DEMANDE DE BREVET EUROPEEN

(21) Numéro de dépôt: 90402950.1

(22) Date de dépôt: 19.10.90

(51) Int. Cl.⁵: **A61B 6/03**, A61B 6/00

(30) Priorité: 24.11.89 FR 8915510

(43) Date de publication de la demande:
**12.06.91 Bulletin 91/24**

(84) Etats contractants désignés:
**DE GB NL**

(71) Demandeur: **GENERAL ELECTRIC CGR S.A.**
**100, rue Camille-Desmoulins**
**F-92130 Issy les Moulineaux(FR)**

(72) Inventeur: **Sirvin, Pierre, Cabinet**
**Ballot-Schmit**
**7, rue Le Sueur**
**F-75116 Paris(FR)**

(74) Mandataire: **Ballot, Paul Denis Jacques et al**
**Cabinet Ballot-Schmit 7, rue le Sueur**
**F-75116 Paris(FR)**

(54) **Appareil de radiologie avec filtre d'homogénéisation.**

(57) L'invention concerne les appareils de radiologie pour examen angiographique.

L'invention réside dans le fait qu'un filtre 20 d'atténuation des rayons X est disposé entre la source 15 de rayonnement X et le patient 14, ledit filtre introduisant, sur le trajet de chaque rayon X, une atténuation telle que l'atténuation totale subie pour ledit rayon sur son trajet jusqu'au récepteur 16 est sensiblement la même pour tous les trajets du faisceau 19 de manière à homogénéiser l'exposition du récepteur formant l'image.

FIG. 1

EP 0 431 989 A1

## APPAREIL DE RADIOLOGIE AVEC FILTRE D'HOMOGENEISATION

L'invention concerne les appareils de radiologie et, plus particulièrement dans ces appareils, un dispositif ou filtre qui permet de modifier spatialement l'exposition au rayonnement X en fonction de la morphologie du corps du patient ou de la partie du corps recevant le rayonnement X.

L'invention est plus particulièrement destinée aux appareils de radiologie qui sont utilisés pour les examens angiographiques des membres inférieurs. Il est rappelé que l'angiographie est la technique radiologique appliquée et adaptée au réseau vasculaire : artères, veines, tissus perfusés.

Elle fait appel à des liquides à base d'iode, dits de contraste, opaques aux rayons X, et injectés dans le réseau vasculaire afin de permettre sa visualisation par différenciation avec les tissus environnants. De manière plus précise, le patient est allongé sur une table qui est prévue de manière à se déplacer sous une source de rayonnement associée à un récepteur disposé de l'autre côté du patient par rapport à la source. Le praticien injecte le liquide opaque aux rayons X, appelé liquide de contraste, dans une artère ou une veine du patient allongé sur la table. Puis, quelques secondes après cette injection, il réalise plusieurs clichés successifs du patient de manière à visualiser et mesurer la progression au cours du temps du produit de contraste dans les vaisseaux sanguins.

Lorsque l'examen angiographique concerne les membres inférieurs, c'est-à-dire une longueur de 120 centimètres environ, plusieurs procédés sont actuellement mis en oeuvre.

Le premier procédé consiste à utiliser comme récepteur un équipement appelé artériophlébographe comportant, sur un tambour hexagonal six couples écran renforçateur/film de 120 cm en longueur par 35 cm en largeur et permettant six irradiations, donc six clichés photographiques à des temps différents. Dans ce procédé, chaque cliché donne une image de la longueur totale des membres et le patient reçoit donc une dose importante de rayonnement X puisque toute la partie basse du corps est exposée à chaque cliché. Par ailleurs, comme l'exposition est la même pour toute la partie basse du corps, il en résulte une grande différence de contraste entre l'abdomen et les pieds du fait des différences d'absorption présentées par ces parties au rayonnement X. Cette grande différence de contraste rend difficile le repérage du produit de contraste.

Pour réduire cette différence de contraste, il a été proposé de placer sur le trajet du faisceau de rayons X un filtre rudimentaire, du type lame d'épaisseur variable en forme de coin, qui affaiblit plus le rayonnement X au niveau des pieds qu'à celui de l'abdomen. Il a également été proposé un écran renforçateur dont le rendement rayons X/photons lumineux est variable sur la longueur des membres inférieurs de manière à présenter un faible rendement au niveau des pieds et un rendement moyen ou fort au niveau de l'abdomen. Les modifications du contraste qui sont obtenues par de tels dispositifs, lame en coin ou écran renforçateur à rendement variable, sont assez grossières.

Un deuxième procédé consiste à examiner les membres inférieurs zone par zone, chaque zone correspondant, par exemple, à un champ utile de 35 cm par 35 cm ou à un diamètre de 30 cm ou 35 cm. Pour cela, on effectue un déplacement relatif du faisceau de rayonnement X et du patient entre chaque pose de manière que chaque pose donne une image partielle des membres et que l'ensemble des images obtenues couvre la totalité de la longueur des membres.

Dans ce deuxième procédé, les paramètres d'exposition varient d'une pose à la suivante pour tenir compte de la variation d'épaisseur entre l'abdomen et les pieds. En pratique, étant donné la dynamique des variations nécessaires, il faut faire varier à la fois le produit mA.s du courant anodique mA du tube de la source de rayonnement X par le temps de pose s et la tension d'alimentation kV du tube; il en résulte alors une modification du contraste des images prises en différentes zones du patient, ce qui nuit au repérage du produit de contraste et, plus généralement, à l'analyse des images, notamment en cas de traitement numérique des images.

Dans l'un ou l'autre procédé qui a été décrit succinctement ci-dessus, certaines parties de la surface sensible du récepteur, correspondant à l'entre-jambes ou aux bords externes des membres, sont soumis au rayonnement X à feu nu, ce qui dégrade la qualité de l'image. Ce problème est résolu par la disposition autour du patient et dans l'espace entre-jambes de divers corps absorbants tels que des cylindres de plastique remplis d'eau. La manipulation de ces cylindres constitue un inconvénient autant pour le patient que pour le praticien.

Le but de la présente invention est donc de réaliser un appareil de radiologie qui ne présente pas les inconvénients précités. Ceci est obtenu par l'utilisation d'un dispositif d'absorption ou de filtrage qui est disposé entre la source de rayonnement et le récepteur et, de préférence, à proximité de la source.

La présente invention concerne un appareil de radiologie, notamment pour l'examen radiologique des membres inférieurs, comportant une source de rayonnement X qui émet un faisceau de rayons X en direction d'un patient, une table sur laquelle le patient peut s'allonger et un récepteur de rayons X disposé du côté de la table opposé à celui ou est placée la source, la table et/ou le couple source-récepteur pouvant se déplacer l'un par rapport à l'autre de manière que le faisceau de rayons X balaye au moins les membres inférieurs du patient dans le sens longitudinal, caractérisé en ce qu'il comprend, en outre, un filtre d'atténuation des rayons X qui est disposé entre la source de rayonnement X et le récepteur, ledit dispositif présentant, suivant chaque trajet de rayon X du faisceau, une atténuation telle que l'atténuation totale subie par ledit rayon sur son trajet jusqu'au récepteur soit sensiblement la même pour tous les trajets du faisceau de manière à homogénéiser l'exposition du récepteur formant l'image. De préférence, le filtre est disposé à proximité de la source de rayonnement et des premiers moyens sont prévus pour le déplacer perpendiculairement par rapport au faisceau de manière que ledit faisceau intercepte les parties concordantes du filtre et du corps du patient.

Selon une autre caractéristique de l'invention, des seconds moyens sont prévus pour modifier le rapport entre la distance source-filtre et/ou celle filtre-patient de manière à adapter les dimensions du filtre à celles du patient.

Selon une autre caractéristique de l'invention, des troisièmes moyens sont prévus pour déplacer horizontalement le filtre sur une distance différente de celle du pas d'avancement défini par le rapport homothétique entre la taille du patient et la longueur du filtre.

Selon une autre caractéristique de l'invention, il est prévu d'utiliser plusieurs filtres interchangeables, chaque filtre étant adapté à la morphologie du patient à examiner.

D'autres caractéristiques et avantages de la présente invention apparaîtront à la lecture de la description suivante d'un exemple particulier de réalisation, ladite description étant faite en relation avec les dessins joints dans lesquels:

- la figure 1 représente schématiquement un appareil de radiologie pour examen angiographique comportant un filtre d'homogénéisation selon la présente invention,
- la figure 2 est un diagramme montrant les relations homothétiques qui permettent de réaliser le filtre d'homogénéisation selon l'invention,
- les figures 3-a et 3-b sont des diagrammes analogues à celui de la figure 2 mais correspondant à des positions différentes du faisceau de rayons X par rapport aux membres inférieurs, et
- la figure 4 est une vue en perspective cavalière d'un filtre d'homogénéisation selon la présente invention.

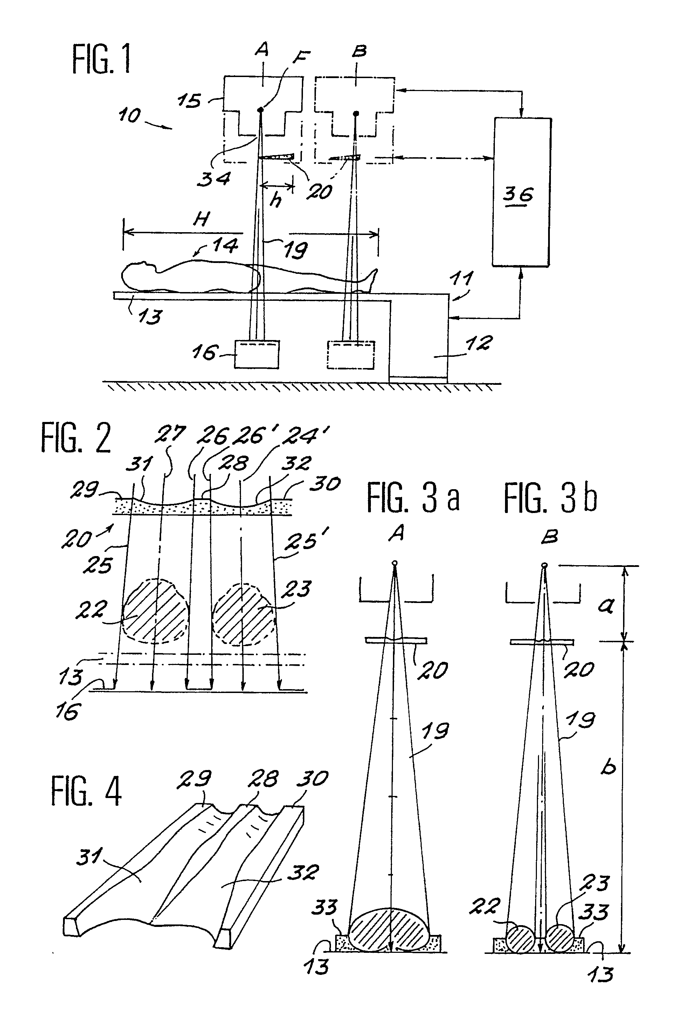

Un appareil de radiologie 10 pour examen angiographique comprend très schématiquement (figure 1) une table 11 comportant un socle 12 sur lequel est monté un panneau coulissant 13 pour supporter un patient 14. Le patient 14 est irradié par un faisceau 19 de rayons X qui est émis par une source de rayonnement 15 de foyer F et d'ouverture 34. Les rayons X, plus ou moins absorbés par le corps du patient, sont détectés par un récepteur 16 qui peut être de tous types connus tels qu'un film photographique, un couple écran-film photographique ou un intensificateur d'image. Dans ce dernier cas, les signaux lumineux qui sont fournis par l'intensificateur d'image sont traités de manière connue pour obtenir des images permettant de suivre la progression du produit de contraste.

Pour suivre cette progression du produit de contraste, le faisceau 19 de rayons X doit être déplacé par rapport au patient 14, soit en déplaçant la source 15 comme cela est représenté sur la figure 1, soit en faisant coulisser le panneau 13. Sur cette figure, les moyens pour commander le déplacement de la source 15 et du récepteur 16 ont été représentés par un dispositif 36. Ce dispositif 36 est en fait un calculateur qui reçoit des informations de position de la source 15 et de la table 11 et qui fournit des instructions de déplacement à la source 15 et à la table 11, ces derniers comportant des moyens de déplacement horizontal motorisé non représentés sur la figure 1.

Comme le montre les figures 2 et 3, les rayons X subissent, sur leur trajet, des atténuations très différentes du fait qu'ils traversent des parties du corps du patient qui ont des épaisseurs très différentes et/ou qui présentent des coefficients d'absorption différents. C'est ainsi que l'abdomen atténuera plus fortement les rayons X que les jambes du fait de la différence d'épaisseur mais que les os des jambes les atténueront également fortement du fait de leur coefficient d'absorption plus élevé par rapport à celui des tissus de l'abdomen. En outre, pour l'entre-jambes et les régions latérales des membres et de l'abdomen, les rayons X ne sont pas atténués. Ces phénomènes conduisent à des images présentant des contrastes très importants qui les rendent difficiles à analyser ou qui obligent le praticien à modifier les paramètres d'exposition.

Pour pallier ces inconvénients, l'invention propose d'interposer entre la source de rayonnement X et le récepteur, de préférence entre la source et le patient et à proximité de la source, un filtre 20 dit d'homogénéisation.

Le filtre 20 est constitué d'une plaque 27 qui est réalisée en un matériau absorbant les rayons X et dont l'épaisseur est variable de manière à introduire, suivant chaque trajet de rayon X du faisceau, une atténuation telle que l'atténuation totale subie par ledit rayon X sur son trajet jusqu'au récepteur soit sensiblement la même pour tous les trajets du faisceau. Ainsi, l'atténuation introduite sera maximale pour la zone de l'entre-jambes, elle sera minimale pour la zone de l'abdomen et aura une valeur intermédiaire pour les autres zones. On obtient alors une image dont l'exposition est homogénéisée.

Une compensation parfaite conduirait à l'obtention d'une image uniformément grise où seul apparaitrait le produit de contraste véhiculé par les vaisseaux sanguins.

Les diagrammes géométriques des figures 2 et 3 permettent de comprendre comment il est possible de déterminer l'épaisseur du filtre pour chaque trajet de rayon X en tenant compte des positions respectives du filtre 20 et du patient 14 par rapport au foyer F de la source 15 de rayonnement X, positions qui définissent un rapport homothétique. Ce rapport homothétique permet de calculer les dimensions latérales et transversales du filtre, dimensions qui sont d'autant plus petites que le filtre est proche du foyer F.

Sur la figure 2, qui correspond à une coupe au niveau des genoux du patient, les rayons 26 et 26′ déterminent les limites de l'entre-jambes et définissent la zone centrale 28 à forte atténuation (ou épaisseur) du filtre. Les rayons 25 et 25′ déterminent les bords latéraux du corps du patient et définissent les zones externes 29 et 30 à forte atténuation (ou épaisseur) du filtre. Enfin, tous les rayons tels que 24 et 24′ sont atténués respectivement par les jambes 22 et 23 du patient et définissent les zones 31 et 32 à atténuation (ou épaisseur) variable.

D'une manière générale, l'épaisseur de la plaque 27 est choisie de manière à introduire, sur le trajet de chaque rayon X, une atténuation telle que l'atténuation totale subit par le rayon X sur son trajet jusqu'au récepteur 16 soit sensiblement la même pour tous les trajets du faisceau.

Sur les figures 3-a et 3-b, qui sont des coupes schématiques de l'appareil de la figure 1 pour les positions respectives A et B du couple source-récepteur (le récepteur n'étant pas représenté), on a montré ce que serait le filtre s'il était placé au niveau du patient: un demi-moule 33 des membres inférieurs du patient.

Selon l'invention, le filtre est placé à proximité du foyer F, à la sortie de la source 15, ce qui, dans le cas. de la figure 3, définit un rapport homothétique de cinq entre les dimensions latérales et transversales du demi-moule 33 et celles du filtre 20. Quant à l'épaisseur du filtre, elle ne dépend pas du rapport homothétique mais de l'atténuation subie par les rayons au niveau du corps du patient ainsi que du coefficient d'absorption du matériau utilisé pour réaliser le filtre.

Comme l'appareil de radiologie 10 est prévu pour effectuer plusieurs clichés des membres inférieurs, chaque cliché correspondant à une zone différente, le filtre 20 doit être déplacé horizontalement devant la source 15 de rayonnement pour mettre en concordance les zones correspondantes du filtre et du patient. A cet effet, le filtre 20 est supporté par la source de rayonnement 15 de manière à pouvoir coulisser par rapport à l'ouverture 34 du diaphragme de la source.

Sur la figure 1, le dispositif de déplacement horizontal du filtre 20 n'a pas été représenté mais il est clair qu'il peut être réalisé de différentes manières sans faire oeuvre d'invention. Ainsi, le filtre peut être motorisé et commandé en synchronisme avec le déplacement horizontal du couple source-récepteur ou celui du panneau 13 dans le cas d'un appareil de radiologie du type à déplacement du patient. Sur la figure 1, les moyens de commande du déplacement horizontal du filtre font partie du dispositif 36 mais les moyens de déplacement motorisé n'ont pas été représentés.

Selon l'invention, le dispositif 36 est également prévu pour commander le déplacement vertical du filtre 20 et/ou de la source 15 et/ou du panneau 13 de manière à faire varier le rapport d'homothétie $K = (a+b)/a$ et ainsi réaliser une adaptation du filtre à la taille du patient. Ces moyens de déplacement vertical n'ont pas été représentés sur la figure 1.

En outre, le dispositif 36 est prévu pour adapter le déplacement horizontal du filtre en fonction du rapport d'homothétie $K$ et du rapport $K' = H/h$ entre la taille $H$ du patient et la longueur $h$ du filtre. Il est à noter que $K = K'$ pour le filtre qui a été réalisé pour le patient-type et le déplacement du filtre entre deux clichés est égal à $h/K$ de manière à obtenir un déplacement $H/K$ sur le patient.

Si, pour adapter le filtre à la taille $H1$ du patient, on modifie $K$ qui devient $K1$, on doit également modifier la longueur du déplacement horizontal du filtre entre deux clichés de manière à maintenir la correspondance à chaque cliché entre les parties correspondantes du filtre et du patient. Cette modification du déplacement est obtenu par le dispositif 36

Le filtre doit être adapté à la morphologie du patient et il est proposé, selon l'invention, de réaliser plusieurs modèles de filtre selon que le patient est du sexe masculin ou féminin, qu'il est de grande, moyenne ou petite taille ou encore en fonction de son poids. Le praticien aura ainsi à sa disposition un jeu de filtres parmi lesquels il choisira celui qui se rapproche le plus du patient à examiner.

Le filtre selon l'invention permet donc d'obtenir une homogénéisation de l'exposition des images et, en conséquence, une meilleure distinction du produit de contraste. De plus, il n'est pas nécessaire de modifier les paramètres d'exposition d'une image à la suivante.

Enfin, une telle image homogénéisée présente une dynamique plus faible et permet un codage numérique sans perte d'information: ceci conduit à un meilleur traitement numérique de l'image.

L'invention a été décrite en plaçant le filtre à la sortie de la source de rayonnement mais il serait préférable de l'intégrer dans l'ensemble constitué du tube à rayons X et du collimateur à iris et/ou à volets. Le matériau dont est constitué le filtre selon l'invention est, par exemple, une résine acrylique chargée en plomb comme par exemple le produit vendu sur l'application de "KYOWA GLASS".

L'atténuation variable du filtre a été obtenue par une épaisseur variable de la plaque 27 mais on peut également faire varier cette atténuation par une composition différente du matériau, par exemple en ajoutant des particules qui sont plus absorbantes, notamment sur les trajets de l'entre-jambes et des bords externes.

Les moyens de déplacement horizontal et vertical de la source 15, du filtre 20 et de la table 11 n'ont pas été représentés sur la figure 1 afin de ne pas la compliquer inutilement. Par ailleurs, ces moyens sont connus de l'homme de métier et peuvent être mis en oeuvre sans faire oeuvre d'invention.

## Revendications

1. Appareil de radiologie, notamment pour l'examen radiologique des membres inférieurs, comportant une source (15) de rayonnement X qui émet un faisceau (19) de rayons X en direction d'un patient (14), une table (11) sur laquelle le patient peut s'allonger, et un récepteur (16) de rayons X disposé du côté de la table opposé à celui où est placée la source (10), la table et/ou le couple source-récepteur pouvant se déplacer l'un par rapport à l'autre de manière que le faisceau (19) de rayons X balaye au moins les membres inférieurs du patient dans le sens longitudinal, caractérisé en ce qu'il comprend en outre, un filtre d'atténuation (20) des rayons X qui est disposé entre la source (15) de rayonnement X et le récepteur (16), ledit filtre présentant, suivant chaque trajet de rayon X du faisceau, une atténuation telle que l'atténuation totale subie par ledit rayon sur son trajet jusqu'au récepteur (16) soit sensiblement la même pour tous les trajets du faisceau de manière à homogénéiser l'exposition du récepteur formant l'image, et en ce que ledit filtre (20) est associé à des moyens (36) pour le déplacer perpendiculairement par rapport au faisceau (19) de manière que ledit faisceau intercepte les parties concordantes du filtre (20) et du corps du patient.

2. Appareil de radiologie selon la revendication 1, caractérisé en ce qu'il est prévu des moyens (36) pour modifier le rapport d'homothétie K = (a + b)/a entre la distance source-patient (a + b) et la distance source-filtre a, de manière à adapter le filtre (20) à la taille du patient.

3. Appareil de radiologie selon la revendication 2, caractérisé en ce que la modification du rapport d'homothéthie est obtenue par des moyens de déplacement vertical du filtre (20).

4. Appareil de radiologie selon la revendication 2, caractérisé en ce que la modification du rapport d'homothétie est obtenue par les moyens de déplacement vertical du panneau (13) du support du patient.

5. Appareil de radiologie selon la revendication 2, caractérisé en ce que la modification du rapport d'homothétie est obtenue par des moyens de déplacement vertical de la source (15).

6. Appareil de radiologie selon l'une quelconque des revendications précédentes 1 à 5, caractérisé en ce qu'il est prévu une pluralité de filtres interchangeables, chaque filtre étant adapté à un type de morphologie du patient.

7. Appareil de radiologie selon la revendication 1 à 6, caractérisé en ce que le filtre est disposé à proximité de la source (15) et en ce que les moyens pour déplacer le filtre (20) sont solidaires de ladite source (15).

8. Appareil de radiologie selon l'une quelconque des revendications précédentes 1 à 7, caractérisé en ce que l'atténuation variable du filtre est obtenue par une modification de l'épaisseur du matériau constituant ledit filtre.

9. Appareil de radiologie selon l'une quelconque des revendications 1 à 7, caractérisé en ce que l'atténuation variable du filtre est obtenue par une modification de la composition du matériau constituant le filtre.

FIG. 1

FIG. 2

FIG. 3 a

FIG. 3 b

FIG. 4

Office européen
des brevets

# RAPPORT DE RECHERCHE EUROPEENNE

Numero de la demande

EP   90 40 2950

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | US-A-3 755 672  (P. EDHOLM et al.) * colonne 2, ligne 54 – colonne 3, ligne 65; colonne 5, lignes 9-33; figures 1,2 * | 1,8 | A 61 B   6/03 A 61 B   6/00 |
| A | | 2,6 | |
| X | FR-A-2 485 790  (COMP. GENERALE DE RADIOLOGIE) * page 2, lignes 10-20; page 3, ligne 18 – page 7, ligne 27; figures 1,2 * | 1 | |
| A | | 6,8 | |
| A | DE-A-3 017 745  (VARIAN ASSOCIATES INC.) * page 6, ligne 20 – page 8, ligne 17; figures 2,3 * | 1,9 | |
| A | EP-A-0 146 992  (N.V. PHILIPS' GLOEILAMPENFABRIEKEN) * page 2, ligne 28 – page 4, ligne 11; figures 1,2 * | 1,6,9 | |

DOMAINES TECHNIQUES RECHERCHES (Int. Cl.5)

A 61 B   6/00
G 21 K   1/00

Le présent rapport a été établi pour toutes les revendications

| Lieu de la recherche | Date d'achèvement de la recherche | Examinateur |
|---|---|---|
| BERLIN | 25-02-1991 | WEIHS J.A. |

EPO FORM 1503 03.82 (P0402)